# EUROPEAN PATENT APPLICATION

(11) **EP 4 501 349 A1**
(43) Date of publication of application: **05.02.2025**
(21) Application number: 23778381.6
(22) Date of filing: 30.03.2023
(51) Int. Cl.: A61K 38/48, A61P 21/02

(54) **BOTULINUM TOXIN PROTEIN COMPOSITION, METHOD FOR PREPARING SAME, AND USE THEREOF**

(30) Priority: 01.04.2022 CN 202210337349
(71) Applicant: Chongqing Claruvis Pharmaceutical Co., Ltd., Chongqing 400713 (CN)
(72) Inventor: YANG, Wu, Chongqing 400713 (CN); GONG, Hui, Chongqing 400713 (CN)
(74) Representative: HGF
(86) International application number: PCT/CN2023/085106
(87) International publication number: WO 2023/186016

(57) **Abstract**

The present invention provides a botulinum toxin protein composition, mainly comprising botulinum toxin protein, a saccharide, a salt, and human serum albumin. The botulinum toxin protein composition of the present invention features good activity and stable properties, and can maintain the activity of the botulinum toxin protein at a high level after long-term storage.

## Description

### TECHNICAL FIELD

The present invention relates to the technical field of cholinergic innervation medicines or medical cosmetology, and in particular, to a botulinum toxin protein composition, a preparation method therefor, and use thereof.

### BACKGROUND

Native botulinum neurotoxins (BoNTs) are toxin proteins produced by an anaerobic bacterium *Clostridium botulinum.* BoNTs inhibit the release of cholinergic neurotransmitters by cleaving membrane proteins associated with vesicles of the acetylcholinergic nerve terminals, resulting in flaccid paralysis of the cholinergically innervated tissues, and have a wide range of applications in the medical and cosmetic fields.

Currently, seven types of BoNTs have been identified, and named as BoNT/A to BoNT/G, respectively. BoNTs have rapidly developed into a class of drugs for the treatment of cholinergic hyperexcitability due to their unique pharmacological properties such as high specificity for the nervous system, limited diffusivity upon local injection, and the reversibility of their effects. Since the 1980s, when the U.S. Food and Drug Administration officially approved BoNTs for clinical treatment, the indications that BoNTs are applicable have been continuously expanding, especially in the field of medical aesthetics, where the growth has been very rapid.

The effects of BoNTs are related to the characteristics of their protein structure, which is divided into three structural regions encoded by the BoNT genes produced by the anaerobic bacterium *Clostridium botulinum* and formed through folding. The three structural regions are respectively a binding region for binding a nerve terminal cell membrane, a transport region for internalizing botulinum toxin into a nerve synapse terminal, and an enzymatic activity region for enzyme digestion of a nerve terminal vesicle-associated membrane protein. *Clostridium botulinum* initially produces a 150 kDa inactive single-chain polypeptide (progenitor toxin), which is cleaved enzymatically to form two peptide segments by an endobacterial protease. The biologically active botulinum toxin is composed of a light chain (L, 50 kDa) and a heavy chain (H, 100 kDa), and the heavy chain and the light chain are linked by a disulfide bond.

BoNTs have the property of binding to nerve terminals, and can cause local muscle paralysis. This property allows BoNTs to be widely used in the field of medical aesthetics. Specifically, BoNTs interact with receptors on the presynaptic membrane of nerve terminals, and then the transport region completes the internalization of BoNTs to form vesicles to enter the cytoplasm of the presynaptic membrane. After BoNTs enter the nerve terminals, the protease of BoNTs has high specificity on a 25 kDa synaptosome-associated protein (SNAP-25) released by neurotransmitters from the presynaptic membrane, and can specifically cleave 9 amino acids at the carboxyl terminal of SNAP-25, resulting in the loss of the function of SNAP-25 in mediating the release of neurotransmitters into synaptic clefts. At the neuromuscular junction, the lack of neurotransmitter release leads to the local muscle paralysis due to the inability of the local muscle to receive innervation from the nervous system.

BoNTs produced by the anaerobic bacterium *Clostridium botulinum* co-express with hemagglutinin proteins and non-hemagglutinin proteins produced within the *Clostridium botulinum* to form a complex. The formation of the complex protects against the attack of botulinum toxin on the human or animal body via the gastrointestinal tract. These non-botulinum toxin proteins do not possess the activity of botulinum toxin, have no therapeutic effect, and do not possess protective effects against botulinum toxin following intramuscular injection. On the contrary, the ingestion of foreign proteins in the formulation upon intramuscular injection of botulinum toxin is increased, thereby increasing the risk of the body's immune response to these foreign proteins. Although there are precedents of extracting purified botulinum toxin proteins from a multi-protein complex expressed by *Clostridium botulinum* in the prior art, obtaining botulinum toxin proteins with high purity by using the aforementioned method has the disadvantages of complex steps, time consumption, low efficiency, and high cost, and fails to solve the biological risks associated with the preservation, fermentation, and amplification of *Clostridium botulinum,* thus imposing significant difficulties in industrial production.

Meanwhile, the preservation of botulinum toxin protein formulations in the prior art still has the problem of poor durability of activity, which leads to the degradation of the activity of the botulinum toxin protein at the time of use, thereby affecting its therapeutic effect.

### SUMMARY

In view of the technical problems put forward in the BACKGROUND, the present invention provides a recombinant botulinum toxin protein composition, a preparation method therefor, and use thereof.

In order to achieve the aforementioned objectives, the present invention adopts the following technical solutions:
The present invention provides a botulinum toxin protein composition, comprising the following components:
a botulinum toxin protein, human serum albumin, a saccharide, and a salt,
wherein preferably, the human serum albumin has a mass percentage in the range of 0.2% to 2%;
preferably, the human serum albumin has a mass percentage in the range of 0.5% to 1%.

Preferably, the saccharide has a mass percentage in the range of 0.5% to 6%;
preferably, the saccharide has a mass percentage in the range of 0.5% to 2%;
more preferably, the saccharide has a mass percentage in the range of 0.5% to 1%;
preferably, the saccharide is selected from one or more of trehalose, sucrose, maltose, fructose, raffinose, lactose, and glucose;
preferably, the saccharide is selected from one or more of sucrose and lactose.

Preferably, the salt has a mass percentage in the range of 0.4% to 1%;
more preferably, the salt has a mass percentage in the range of 0.4% to 0.9%;
particularly preferably, the salt has a mass percentage in the range of 0.45% to 0.9%.

Preferably, the salt is selected from any one of an inorganic salt or an organic salt;
more preferably, the inorganic salt is selected from one or more of sodium chloride, sodium phosphate, magnesium sulfate, sodium acetate, sodium propionate, and tripotassium phosphate;
more preferably, the organic salt is selected from one or more of sodium lactate and sodium succinate.

In one embodiment of the present invention, the inorganic salt is sodium chloride.

It should be noted that in the present invention, the amount of the botulinum toxin protein can be determined according to the toxic potency of the botulinum toxin protein, the purpose of use, and the weight of the target.

Preferably, the botulinum toxin protein is a recombinant botulinum toxin protein. The biological risks in the production process can be effectively reduced.

Preferably, the composition comprises a botulinum toxin protein, human serum albumin, sucrose, and sodium chloride.

Preferably, the composition comprises the following components:
the human serum albumin having a mass percentage in the range of 0.2% to 2%,
the sucrose having a mass percentage in the range of 0.5% to 6%,
the sodium chloride having a mass percentage in the range of 0.4% to 1%, and
the balance being water.

More preferably, the human serum albumin has a mass percentage in the range of 0.5% to 1%;
the sucrose has a mass percentage in the range of 0.5% to 2%;
the sodium chloride has a mass percentage in the range of 0.4% to 0.9%;
the balance is water.

Particularly preferably, the human serum albumin has a mass percentage in the range of 0.5% to 1%;
the sucrose has a mass percentage in the range of 0.5% to 1%;
the sodium chloride has a mass percentage in the range of 0.45% to 0.9%;
the balance is water.

Preferably, the recombinant botulinum toxin protein has at least one dimeric structure composed of polypeptides formed from a single-chain polypeptide cleaved by a protease,
wherein the single-chain polypeptide comprises:
(I) a first polypeptide fragment comprising:
   (a) a tag protein,
   (b) a structural region comprising a first protease cleavage site; and
   (c) a short linker peptide; and
(II) a second polypeptide fragment comprising:
   (d) a first functional amino acid structural region comprising a metal ion-dependent protease activity domain;
   (e) a structural region comprising a second protease cleavage site; and
   (f) a second functional amino acid structural region comprising a receptor-binding domain capable of binding to a surface receptor of a target cell and/or a translocation domain capable of mediating the transfer of the polypeptide across a vesicle membrane.

The term "tag protein" refers to a class of protein molecules that can bind to a specific ligand.

Preferably, the tag protein is selected from a tag protein known to those skilled in the art or a tag protein designed by a computer program, and is capable of specifically binding to a known substrate.

More preferably, the tag protein is selected from, but is not limited to, the following proteins: glutathione S-transferase (GST), C-myc, chitin-binding domain, maltose-binding protein (MBP), SUMO heterologous affinity moiety, monoclonal antibody or protein A, streptavidin-binding protein (SBP), cellulose-binding domain, calmodulin-binding peptide, S tag, Strep tag II, FLA, protein A, protein G, histidine affinity tag (HAT), and polyhistidine.

Preferably, the "tag protein" can increase the solubility of a toxin polypeptide precursor in a host.

Preferably, the "tag protein" allows the presence of a toxin polypeptide precursor in a host cell in a soluble manner.

In one specific embodiment, the tag protein is located at the N terminus of the single-chain polypeptide.

In one specific embodiment, the tag protein is a glutathione S-transferase (GST), and more preferably, the amino acid sequence of the glutathione S-transferase is set forth in SEQ ID NO: 2.

Preferably, neither the first protease cleavage site nor the second protease cleavage site can be cleaved by a human protease or a protease produced by the host cell expressing the single-chain polypeptide.

More preferably, the first protease cleavage site and the second protease cleavage site are identical or different.

The first protease cleavage site and the second protease cleavage site are not recognized and cleaved by an endogenous protease of a host cell expressing same or an organism receiving same.

More preferably, the specific protease is selected from, but is not limited to, one of or a combination of two of the following proteases: a non-human enterokinase, a tobacco etch virus protease, a protease derived from *Bacillus subtilis,* a protease derived from *Bacillus amyloliquefaciens,* a protease derived from rhinovirus, papain, a homologue of papain of an insect, or a homologue of papain of a crustacean.

In one specific embodiment, the protease that specifically recognizes the first protease and the protease that specifically recognizes the second protease cleavage site are both derived from the protease of rhinovirus.

Preferably, the second enzyme cleavage site is embedded into, partially replaces, or completely replaces a natural loop region between a first functional peptide fragment and a second functional peptide fragment. The embedding refers to an embedding between two certain amino acids in the loop region; the partial replacement refers to the second enzyme cleavage site replacing part of the amino acid sequence of the loop region; the complete replacement refers to the amino acid sequence of the natural loop region being completely replaced by the second enzyme cleavage site.

More preferably, the structural region comprising the first protease cleavage site and the structural region comprising the second protease cleavage site are selected from, but are not limited to, one of or a combination of two of the following enzyme cleavage sites: DDDDK (SEQ ID NO: 12), EXXYXQS/G (wherein EXXYXQS is SEQ ID NO: 13; EXXYXQG is SEQ ID NO: 14), HY, YH, or LEVLFQGP (SEQ ID NO: 4).

In one specific embodiment, the structural region comprising the first protease cleavage site and the structural region comprising the second protease cleavage site are both LEVLFQGP (SEQ ID NO: 4), and the cleavage sites are between Q-G.

Preferably, the short linker peptide has no more than 5 amino acids.

Preferably, the short linker peptide enables the first protease cleavage site to be more easily recognized or bound to by a protease thereof.

More preferably, the short linker peptide does not affect the function of the single-chain polypeptide.

More preferably, the short linker peptide retained at the N terminus of the second polypeptide fragment does not affect the function of the second polypeptide fragment after the cleavage of the first protease cleavage site.

More preferably, a GS part of the short linker peptide has no more than 5 amino acid residues, and even more preferably, the short linker peptide is selected from a glycine-serine (GS) short peptide, GGS,GGGS (SEQ ID NO: 16), GGGGS (SEQ ID NO: 17), GSGS (SEQ ID NO: 18), GGSGS (SEQ ID NO: 19), GSGGS (SEQ ID NO: 20), GGGSS (SEQ ID NO: 22), and other short linker peptides.

In one specific embodiment, the amino acid sequence of the structural region comprising the first protease cleavage site and the short linker peptide is LEVLFQGPLGS (SEQ ID NO: 15).

Preferably, the metal ion-dependent protease activity domain is a Zn²⁺-dependent protease activity domain.

Preferably, the first functional amino acid structural region and/or the second functional amino acid structural region are encoded by a natural sequence and/or an artificially synthesized sequence. More preferably, the first functional amino acid structural region of the single-chain polypeptide comprises a Zn²⁺ protease activity domain of the light chain of a clostridial neurotoxin.

Preferably, a target cell of the receptor-binding domain capable of binding to an antigen on the surface of a human cell in the second functional amino acid structural region refers to a target cell with an SNARE complex, for example: a nerve cell, a pancreatic cell, or other cells with an SNARE complex.

More preferably, the target cell of the second functional amino acid structural region refers to a human nerve cell or a pancreatic cell, and the receptor-binding domain is a receptor-binding domain capable of specifically binding to the human nerve cell or the pancreatic cell.

More preferably, the receptor-binding domain of the second functional amino acid structural region is a cell surface antigen-binding domain of the heavy chain of clostridial neurotoxin, and the translocation domain of the second functional amino acid structural region that mediates the transfer of the polypeptide across the vesicle membrane is a domain that mediates the transfer of clostridial neurotoxin across the vesicle membrane.

More preferably, the clostridial neurotoxin is a botulinum neurotoxin or tetanus toxin. More preferably, the botulinum neurotoxin is selected from any one of serotypes BoNT/A-BoNT/H and derivatives thereof known in the art.

More preferably, the clostridial neurotoxin is selected from any one of tetanus toxin or a derivative thereof. More preferably, the first functional amino acid structural region comprises part or all of the light chain of BoNT/A, BoNT/B, BoNT/C, BoNT/D, BoNT/E, BoNT/F, BoNT/G, BoNT/H, or tetanus toxin.

More preferably, the second functional amino acid structural region comprises part or all of the heavy chain of BoNT/A, BoNT/B, BoNT/C, BoNT/D, BoNT/E, BoNT/F, BoNT/G, BoNT/H, or tetanus toxin.

In one specific embodiment, the first functional amino acid structural region is the light chain of BoNT/A, and the second functional amino acid structural region is the heavy chain of BoNT/A.

More preferably, the first functional amino acid structural region and the second functional amino acid structural region may be from different serotypes. The first functional amino acid structural region and the second functional amino acid structural region may be any combination of the aforementioned serotypes. For example, the first functional amino acid structural region is derived from the light chain of BoNT/A and the second functional amino acid structural region is derived from the heavy chain of BoNT/B, or the first functional amino acid structural region is derived from the light chain of BoNT/A and the second functional amino acid structural region is derived from the heavy chain of BoNT/C.

More preferably, the second polypeptide fragment has an amino acid sequence comprising a fragment set forth in SEQ ID NO: 7.

In one specific embodiment, the single-chain polypeptide sequentially comprises, from the N terminus, a glutathione S-transferase, LEVLFQGPLGS (SEQ ID NO: 15), the light chain of BoNT/A, LEVLFQGP (SEQ ID NO: 4), and the heavy chain of BoNT/A. More preferably, the single-chain polypeptide has an amino acid sequence set forth in SEQ ID NO: 11.

More preferably, the recombinant botulinum toxin protein is selected from a recombinant botulinum toxin type A protein, and is prepared by cleaving a single-chain polypeptide with a first protease to remove a tag protein, and forming at least one dimer with the first functional amino acid structural region and the second functional amino acid structural region after cleavage with a second protease. The immune risk of the ingestion of inactive botulinum neurotoxin proteins into the human body in the process of extracting botulinum toxin from the traditional *Clostridium botulinum* can be fundamentally avoided, and the biological risk in the production process can be effectively reduced.

Preferably, the first functional amino acid structural region and the second functional amino acid structural region are linked to form a dimeric structure through a disulfide bond.

The recombinant botulinum toxin type A protein precursor of the present invention is formed from a specific single-chain polypeptide. The related single-chain polypeptide has a specific structure, and the single-chain polypeptide with the structure is characterized by strong specificity and low toxicity. Specifically, from the perspective of strong specificity, the tag protein in the single-chain polypeptide can bind to a specific ligand and subsequently be separated from the ligand, so that the molecule co-expressed with the tag protein can be selected from a variety of polypeptides or protein products more easily; in the preparation process, the purity of the product can be remarkably improved, thus giving a higher product yield and purity. From the perspective of low toxicity, the tag protein in the single-chain polypeptide prevents the protease structural domain of the first functional amino acid structural region in the polypeptide from exerting its protease functionality; by replacing the natural enzyme cleavage site between the first and second functional amino acid structural regions with a site recognized only by a specific enzyme, the molecular activity (neurotoxicity) of the single-chain polypeptide is reduced to one ten-thousandth or less that of the natural toxin, thereby effectively reducing the toxicity; due to the greatly reduced toxicity, the safety of the production process is improved, making the entire industrial production easier to operate. That is, the single-chain polypeptide in the recombinant botulinum toxin type A protein related to the present invention is easier for industrial production and purification due to the higher yield, purity, and safety as compared with the prior art.

As known in the prior art, botulinum toxin products on the market are all extracted from *Clostridium botulinum,* which itself is pathogenic and falls within the category of biochemical weapons. Therefore, there are certain risks associated with the preservation, fermentation, and amplification of *Clostridium botulinum* in the extraction and preparation of botulinum toxin from *Clostridium botulinum.* Unlike natural type A botulinum toxin, the recombinant botulinum toxin protein provided herein is expressed from non-pathogenic *Escherichia coli,* which not only reduces the biological risks in the production process, but also makes the preparation method for recombinant proteins simple, safe, efficient, and stable.

The present invention further provides a preparation method for a botulinum toxin protein composition, comprising mixing the aforementioned components with water and then lyophilizing to obtain the composition;
preferably, the aforementioned preparation method comprises:
(a) preparing and purifying a botulinum toxin protein;
(b) adding human serum albumin, a saccharide, a salt, and water to the botulinum toxin protein; and
(c) lyophilizing.

The water is water for injection or normal saline.

The botulinum toxin protein, the human serum albumin, the saccharide, and/or the salt are as defined above.

In one specific embodiment, the preparation method comprises:
(a) preparing and purifying a recombinant botulinum toxin type A protein;
(b) adding human serum albumin, sucrose, sodium chloride, and water to the recombinant botulinum toxin type A protein; and
(c) lyophilizing.

Alternatively,
the water is water for injection or normal saline.

The recombinant botulinum toxin type A protein composition is in the form of a lyophilized formulation.

The present invention further provides a botulinum toxin protein lyophilized formulation prepared by the aforementioned preparation method.

The present invention further provides a cosmetic method comprising administrating any one of the compositions described herein.

Preferably, the cosmetic method described herein can be for therapeutic or non-therapeutic purposes.

Preferably, the cosmetic method comprises wrinkle smoothing or prevention, facial slimming, body contouring, and scar repair.

The present invention further provides a method for preventing and/or treating a disease associated with cholinergic nerves innervating muscles or exocrine glands, wherein the method comprises administering any one of the compositions described herein.

The present invention further provides use of a botulinum toxin protein composition in preparing a medicament for preventing and/or treating a disease associated with cholinergic nerves innervating muscles or exocrine glands,
or
in preparing a medicament for medical cosmetology,
wherein preferably, the disease associated with cholinergic nerves described herein is selected from:
   one or more of Frey's syndrome, crocodile tears syndrome, armpit hyperhidrosis, plantar hyperhidrosis, head and neck hyperhidrosis, body hyperhidrosis, rhinorrhea, Parkinson's disease, amyotrophic lateral sclerosis, sialorrhea, drooling, salivation, spasticity, palatal myoclonus, myoclonus, myokymia, stiffness, benign myospasm, hereditary chin tremor, abnormal mandibular muscle activity, hemimasticatory spasm, hypertrophic branchial myopathy, masseter hypertrophy, tibialis anterior muscle hypertrophy, nystagmus, oscillating vision, supranuclear gaze palsy, epilepsia partialis continua, planned spasmodic torticollis surgery, abductor vocal cord paralysis, refractory mutational dysphonia, upper esophageal sphincter dysfunction, vocal cord granuloma, stuttering, Tourette syndrome, middle ear myoclonus, protective laryngeal closure, speech failure after laryngectomy, protective ptosis, entropion, sphincter of Oddi dysfunction, pseudoachalasia, non-achalasia esophageal dyskinesia, vaginismus, post-operative bed rest, tremor, bladder dysfunction, hemifacial spasm, reinnervation dyskinesia, stiff person syndrome, tetanus, prostatic hyperplasia, obesity treatment, infantile cerebral palsy, achalasia, anal fissure, allergic rhinitis, depression, dental disease, and neuropathic pain.

The medical cosmetology comprises wrinkle smoothing or prevention, facial slimming, body contouring, and scar repair.

The wrinkle may be viewed as folds, ridges, or creases in the skin. Wrinkles visible in the human face are preferred. Examples of wrinkles include lacrimal furrows, glabellar lines, crow's feet, buccal commissure lines, mandibular lines, perioral wrinkles, buccal folds, marionette lines, cheilogramma, forehead wrinkles, frown lines, bunny lines, nasolabial folds, infraocular wrinkles, and chin folds.

Compared with the prior art, the present invention has the following beneficial effects:
(1) The present invention provides a botulinum toxin protein composition, which, by adopting a specific combination and a specific component proportion of albumin, saccharides, and salts, has a long preservation period, and not only maintains the activity of the botulinum toxin protein but is also particularly suitable for a lyophilized formulation. Moreover, after recovery from lyophilization, the composition can still effectively maintain the activity of the botulinum toxin protein with good therapeutic effects, which effectively addresses the problem of poor activity of botulinum toxin proteins after reconstitution in the prior art.
(2) The preferred technical solution of the present invention also adopts a high-purity and low-toxicity botulinum toxin protein precursor prepared from a specific single-chain polypeptide. Particularly, the recombinant botulinum toxin type A protein composition provided by this preferred technical solution not only significantly improves the purity of the botulinum toxin protein but also fundamentally avoids the immune risk of the ingestion of inactive botulinum neurotoxin proteins into the human body in the process of extracting botulinum toxin from the traditional *Clostridium botulinum.* The biological risk in the production process is effectively reduced. Additionally, the preparation method for the recombinant protein composition of the present invention is simple, safe, efficient, and stable, providing technical support for expanding the application range of botulinum toxin proteins and the large-scale industrial production of toxin protein products.

### BRIEF DESCRIPTION OF THE DRAWINGS

FIG. 1 illustrates the SDS-PAGE results of the preliminary purification of GSTs-BoNT/A and the further removal of GSTs tag, wherein lane 1 is the GSTs-BoNT/A obtained in the preliminary purification by a GST affinity chromatographic column, and lane 2 is BoNT/A without GSTs obtained by removing the GSTs tag protein through the digestion of the preliminarily purified protein with Rinovirus 3C Protease.
FIG. 2 illustrates the SDS-PAGE results of a high-purity BoNT/A protein obtained by further purifying the product with the GSTs tag removed through an ion exchange column chromatography.
FIG. 3 illustrates the verification results of the dissociation of two chains of BoNT/A under reducing conditions.
FIGs. 4-6 illustrate the appearance of lyophilized formulations of the formulation compositions with serial Nos. 1-3 in Example 5 of the present invention.

### DETAILED DESCRIPTION

Unless otherwise defined, all scientific and technical terms used in the present invention have the same meaning as commonly understood by those skilled in the art to which the present invention relates.

In the present invention, the term "U" refers to an international unit, which is commonly used for measuring botulinum toxin, and used in pharmaceutics for representing the diversity of biological abilities. Since the chemical composition of certain pharmaceuticals is not constant or their quality specifications cannot yet be determined by physicochemical methods, the potency of such pharmaceuticals is often determined by biological assays and comparisons with standards. By this bioassay, the smallest potency unit with a certain biological potency is called the "unit" (U). The mouse unit (U) in the present invention represents the amount of the recombinant botulinum toxin type A protein required for intraperitoneal injection to kill 50% of the mice.

The term "BoNT/A" refers to the botulinum toxin type A protein.

The term "tag protein" refers to a polypeptide or protein that is fused to and expressed with a target protein using *in-vitro* DNA recombinant technology, so as to facilitate the expression and detection of the target protein.

The term "human serum albumin", abbreviated as HSA, refers to a protein in human plasma. HSA is capable of transporting fatty acids, bilirubin, amino acids, steroid hormones, metal ions, and many therapeutic molecules in body fluids, and the like. Additionally, it plays a role in maintaining the normal osmotic pressure of the blood. HSA can be used clinically to treat shock and burns, to supplement blood loss due to surgery, accidents, or hemorrhage, and as a plasma expander.

The term "dimeric structure" refers to a macromolecular complex composed of two molecules, often bonded by non-covalent bonds.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

### Example 1 Preparation of Recombinant Botulinum Toxin Type A Protein

Specifically, the process comprises the following 8 steps:

### 1. Design and construction of nucleic acid molecule encoding modified single-chain polypeptide of botulinum toxin:

The nucleic acid molecule comprises the following sequences sequentially from the 5' end:
(a) a nucleotide sequence encoding a glutathione S-transferase set forth in SEQ ID NO: 1, wherein the glutathione S-transferase encoded by the nucleotide sequence has an amino acid sequence set forth in SEQ ID NO: 2;
(b) a nucleotide sequence encoding a first protease cleavage site set forth in SEQ ID NO: 3, wherein the amino acid sequence encoded by the nucleotide sequence is set forth in SEQ ID NO: 4;
(c) a nucleotide sequence encoding a GS short linker peptide is set forth in SEQ ID NO: 5, which is GGATCC; and
(d) a nucleotide sequence encoding a second polypeptide fragment comprising nucleotide sequences of the light chain of BoNT/A, a second protease cleavage site, and the heavy chain of BoNT/A, as set forth in SEQ ID NO: 6, wherein the amino acid sequence encoded by the nucleotide sequence is set forth in SEQ ID NO: 7, the nucleotide sequence encoding the second protease cleavage site is set forth in SEQ ID NO: 8, and the amino acid sequence encoded by the nucleotide sequence is set forth in SEQ ID NO: 9.

There may also be no natural loop region between the light chain of BoNT/A and the heavy chain of BoNT/A. For example, the KTKSLDKGYNK linker sequence as set forth in SEQ ID NO: 21 between the light chain and the heavy chain may be removed to reduce non-specific protease cleavage.

The nucleotide sequence encoding the single-chain polypeptide is set forth in SEQ ID NO: 10, and the amino acid sequence of the encoded single-chain polypeptide is set forth in SEQ ID NO: 11.

### 2. Construction of plasmid comprising nucleic acid molecule of sequence of step 1

The genetically optimized GSTs-BoNT/A was artificially synthesized as described above, and NdeI and NotI enzyme cleavage sites were synthesized and added at the two ends thereof. The GSTs-BoNT/A was digested with the NdeI and the NotI at 37 °C (New England Biolabs), purified by using a QIquick gel extraction kit (Qiagen), and inserted into the NdeI and NotI sites in a pET28a plasmid vector (Novagen) by using a T4 DNAligase (NEB).

### 3. Transfection of host cells with plasmid constructed in step 2

(a) Preparation of competent cells: A tube of *E.coli* BL21 DE3 (New England Biolabs) was inoculated into a test tube containing 3 mL of LB culture medium and cultured with shaking at 37 °C overnight. On the next day, 0.5 mL of the bacterial solution was inoculated into a 250-mL flask containing 50 mL of LB culture medium and cultured with vigorous shaking (250 rpm) at 37 °C for about 3-5 h. When the OD value of the bacterial colony reached 0.3-0.4 at 600 nm, the flask was transferred to an ice bath and incubated for 10-15 min. The bacterial solution was poured into a 50-mL centrifuge tube in an aseptic condition. The solution was centrifuged at 1000 g at 4 °C for 10 min. The supernatant was discarded, and the cells were collected. 10 mL of 0.1 M CaCl₂ was added into the centrifuge tube, and the mixture was mixed well with shaking to resuspend the bacterial cells. Subsequently, the cells were subjected to an ice bath for 30 min, and centrifuged at 1000 g at 4 °C for 10 min. The supernatant was discarded, and 4 mL of 0.1 M CaCl₂ pre-cooled with ice was added to resuspend the collected bacterial cells. The cells were aliquoted at 0.2 mL/tube and preserved at 4 °C for later use within 24 h, and the remaining samples were preserved in a low-temperature freezer at -70 °C.
(b) Transfection: Appropriate amounts of DNA (ng) and competent E. *coli* cells were mixed, co-incubated in an ice bath for 15 min, heat-shocked at 42 °C for 30 s, incubated in an ice bath for 5 min, shaken at 250 rpm for 1 h in a SOC medium, smeared on a plate with antibiotics, and cultured at 37 °C overnight.

### 4. Culture of host cells and induction of single-chain polypeptide expression

Culture medium composition: 11.8 g/L of tryptone, 23.6 g/L of yeast extract, 9.4 g/L of K₂HPO₄, 2.2 g/L of KH₂PO₄, and 4 mL/L of glycerol.

Culture condition: The cells were cultured with shaking at 250 rpm at 37 °C overnight. Expression induction: 1 mM IPTG was added to induce the expression at 25 °C for 5 h when the *E. coli* cells grew to a stage in which the OD600 = 1. The OD600 threshold may be selected from 0.2-1.5, the temperature threshold may be selected from 37 °C to 10 °C, and the expression time may be selected from 5-16 h.

Harvesting cells: *E. coli* cells were collected by centrifugation at 3000 rpm for 30 min.

### 5. Identification of expression level of single-chain polypeptide

A glutathione purification resin chromatographic column having a diameter of 1 cm and a height of 1 cm was prepared using Genscript glutathione purification resin, and 25 mL of the lysate was slowly poured into the upper layer of the chromatographic column (be careful not to agitate the resin), and slowly directed through the column to adsorb GSTs-BoNT/A in the lysate for one hour. The GSTs-BoNT/A was eluted according to a conventional method.

The conventional method is as follows: A chromatographic column was washed with 20 column volumes of a phosphate buffer. GSTs-BoNT/A was eluted with 10 column volumes of a freshly prepared 10 mM glutathione eluent buffer (0.154 g reduced glutathione dissolved in 50 mL of 50 mM Tris-HCl (pH 8.0)) The elution of the fusion protein was monitored by means of the absorbance reading at 280 nm. GSTs tag protein of the eluted protein was cleaved under the effect of Rinovirus 3C Protease.

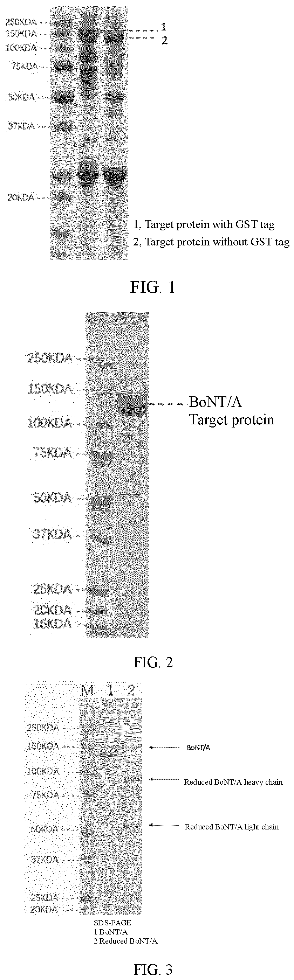

The products before and after the removal of the GSTs tag protein were subjected to a conventional SDS-PAGE experiment. As shown in FIG. 1, compared with lane 1 without tag protein removal, the GSTs tag protein of lane 1 was cleaved to give a BoNT/A molecule without GSTs under the effect of Rinovirus 3C Protease.

Determination of the proportion of GSTs-BoNT/A in total protein: The purified GSTs-BoNT/A were electrophoretically separated at 200 volts by using 4-12% SDS-PAGE (Biorad), and a major band with a molecular weight of 175 kd was GSTs-BoNT/A.

### 6. Removal of tag protein and purification

The product in step 5 was subjected to the removal of GSTs tag protein and the purification of the toxic polypeptide:
The GSTs-BoNT/A re-adsorbed on the glutathione purification resin chromatographic column was treated using Genscript 3C enzyme. The first enzyme cleavage site between the GSTs and BoNT/A was cleaved under the effect of the 3C enzyme. GSTs were separated. The second enzyme cleavage site between the light chain and the heavy chain of BoNT/A was then cleaved.

The glutathione purification resin chromatographic column was treated with a phosphate buffer. The light chain and the heavy chain of BoNT/A were eluted by the phosphate buffer, while the GSTs tag protein retained on the column was thus removed. The product after the removal of GSTs was subjected to a conventional SDS-PAGE experiment. As shown in FIG. 2, the obtained bands did not contain the GSTs tag protein, indicating that the GSTs tag protein had been completely removed. The SDS-PAGE results were scanned, and the purity of the obtained BoNT/A was 90% or more by calculation based on the grey density of the band.

GSS, GSGS (SEQ ID NO: 18), and GGSGS (SEQ ID NO: 19) polypeptides were adopted to replace the GS part of the short linker peptide, which demonstrated a similar effect to that of GS. The tag protein can be well exposed and thus completely cleaved.

### 7. Dissociation of two chains of dimer BoNT/A under reducing conditions

The products of step 6 were subjected to a reduction experiment:
The sample was treated by using 100 mM dithiothreitol at 100 °C for 5 min to reduce the sample, and electrophoretically separated at 200 volts by using 4-12% SDS-PAGE (Biorad) to separate the heavy chain and the light chain. As shown in FIG. 3, the product obtained in step 6 was reduced under reducing conditions, and the product obtained was subjected to a conventional SDS-PAGE experiment to give two different bands having molecular weights of 100 kDa and 50 kDa, respectively, suggesting that the product formed in step 6 was a dimeric structure composed of two peptide fragments linked by a disulfide bond.

### 8. Toxicity assay of GSTs-BoNT/A

The GSTs-BoNT/A obtained in step 5 exhibited an LD₅₀ of 45-450 ng after intraperitoneal injection in mice; considering the purity of the injected botulinum toxin protein, the converted LD₅₀ was 22.5-225 ng, with a median of 123.75 ng. The BoNT/A obtained in step 5 exhibited an LD₅₀ of 0.02-0.05 ng after intraperitoneal injection in mice. Considering the purity of the injected botulinum toxin protein, the converted LD₅₀ was 0.006-0.015 ng, with a median of 0.0105 ng (See Table 1).

**Table 1. Toxicity comparison of GSTs-BoNT/A and BoNT/A molecules in mouse biotoxicity study**

| Test sample | Dose (ng) | Lethality rate in mice at 72 h post-dose (%) |
|---|---|---|
| GST-BoNT/A (50% purity) | 450 | 100 |
| | 45 | 50 |
| | 4.5 | 0 |
| | 0.45 | 0 |
| BoNT/A (30% purity) | 0.05 | 100 |
| | 0.02 | 0 |
| | 0.01 | 0 |
| | 0.005 | 0 |

The GSTs-BoNT/A exhibited the activity of botulinum toxin, and the median lethal dose (LD₅₀) thereof was approximately 11786 times higher than the LD₅₀ of the BoNT/A protein after intraperitoneal injection in mice, indicating that the activity of the toxin polypeptide precursor molecule of the GSTs-BoNT/A recombinant protein is approximately 11786 times weaker than that of the final product BoNT/A. It can be seen that the toxin polypeptide precursor molecule of the GSTs-BoNT/A recombinant protein has high purity and low toxicity, and possesses the activity of botulinum toxin, thereby effectively improving the purity of the botulinum toxin protein. Meanwhile, the recombinant botulinum toxin type A protein prepared in this example does not contain inactive botulinum neurotoxin proteins, thereby fundamentally avoiding the immune risk in the human body, effectively reducing the biological risk in the production process, and providing technical support for expanding the application range of botulinum toxin proteins and the large-scale industrial production of toxin protein products.

### Example 2

### 1. Preparation of lyophilized formulations

For mice, 2 U of the recombinant botulinum toxin type A protein prepared in Example 1 was selected, and then human serum albumin, sucrose, sodium chloride, and water were added to the recombinant botulinum toxin type A protein according to the lyophilization formula (serial Nos. 1-5) listed in Table 2. Subsequently, lyophilization was performed. It can be seen that the preparation method for the recombinant protein composition of the present application is simple, safe, and efficient.

It should be noted that the amount of the recombinant botulinum toxin type A protein in the formula of this example was a therapeutically effective amount selected based on the toxic potency of the recombinant botulinum toxin type A protein and the weight of the mice.

### 2. Reconstitution of lyophilized formulations

Water for injection or normal saline (see Table 2 for details) was added to the series of lyophilized formulations prepared in step 1, and the lyophilized formulations prepared in step 1 were reconstituted.

### 3. Activity assay of lyophilized formulations after reconstitution

### 3.1 Species and grouping

Adult CD-1 mice with an equal number of male and female mice were selected, grouped with 10 mice in each group, weighed, and numbered. The mice in each group were guaranteed to be equal in number and similar in weight.

### 3.2 Administration

Before the administration, each mouse was weighed once (before the administration on the first day) and recorded. The lyophilized formulations reconstituted in step 2 were administered to the mice. During the administration, 0.5 mL of the lyophilized recombinant botulinum toxin type A protein formulations of different formulas were administered to the CD-1 mice by a single intraperitoneal injection. The recombinant botulinum toxin type A protein exhibited an LD₅₀ ranging from 12 ± 3 pg after intraperitoneal injection in mice. After the administration, the weight was measured once every 24 h, and the mortality was determined 24 h (±1 h), 48 h (±1 h), 72 h (±1 h), and 96 h (±1 h) post-dose. The results are shown in Table 2.

The "drug activity" refers to the lethality of mice injected intraperitoneally with the botulinum toxin protein composition.

### 3.3 Analysis of results

As can be seen from Table 2, except for the assay of serial No. 1, other lyophilized formulation compositions showed a drug activity of 70% or more after reconstitution, and the drug activity of serial No. 2 was up to 90%. It can be seen that the components used in serial Nos. 2-5 listed in Table 2 of this example can effectively maintain the activity of the botulinum toxin protein, and the reconstituted lyophilized formulation samples still maintain strong botulinum toxin protein activity, which effectively solves the problem of reduced activity of botulinum toxin proteins after reconstitution in the prior art, and therefore indicates that the botulinum toxin protein composition of the present invention has a good therapeutic effect.

In addition, in this example, it is also unexpectedly found that the content of sodium chloride has a great impact on the drug activity of the lyophilized formulation of the composition of the present invention. Compared to the results of serial Nos. 1-3, it can be seen that the drug activity of the composition without sodium chloride after reconstitution was much lower than that of the composition with sodium chloride. Compared to serial Nos. 2 and 3, and serial Nos. 4 and 5, respectively, it can be seen that when the sucrose content in the composition formula was low and the reconstitution was performed with normal saline (sodium chloride content was 0.9%), the drug activity of the composition was greatly improved, while when the sucrose content was high, there was no significant effect on the drug activity whether normal saline or water for injection was used for reconstitution. An isotonic salt concentration after the reconstitution was preferred to avoid causing undesirable irritation (such as pain) at the injection site.

**Table 2. Different composition formulas used in Example 2 and results of drug activity**

| Serial No. | Recombinant botulinum toxin type A protein prepared in Example 1 | Lyophilization formula | | | | | |
|---|---|---|---|---|---|---|---|
| | | HSA (%) | Sucrose (%) | Sodium chloride (%) | Lyophilization volume (mL) | Reconstitution | Drug activity* (%) |
| 1 | 2U | 0.2 | 0.94 | 0 | 0.5 | Normal saline | 0 |
| 2 | 2U | 0.2 | 0.94 | 0.9 | 0.5 | Normal saline | 90 |
| 3 | 2U | 0.2 | 0.94 | 0.9 | 0.5 | Water for injection | 70 |
| 4 | 2U | 1 | 4.7 | 0.9 | 0.1 | Normal saline | 70 |
| 5 | 2U | 1 | 4.7 | 0.9 | 0.1 | Water for injection | 70 |

### Example 3

In this example, the recombinant botulinum toxin type A protein prepared in Example 1 and the component formulas as shown in Table 3 were used, and the same methods for the preparation, reconstitution, and activity assay of lyophilized formulations as in Example 2 were adopted.

In this example, the drug activity of the recombinant botulinum toxin type A protein at 2 U and 1.54 U under different formula systems as shown in Table 3 was investigated, all of which reached 70% or more. Particularly, when HSA at 1%, sucrose at 1%, sodium chloride at 0.8-0.9%, and recombinant botulinum toxin type A protein at 2 U and 1.54 U were selected for the lyophilization formula, the drug activity after reconstitution was up to 90%, even 100%, indicating that the composition formulas of this example have extremely strong drug activity.

**Table 3. Different composition formulas used in Example 3 and results of drug activity**

| Serial No. | Lyophilization formula | | | Drug activity | |
|---|---|---|---|---|---|
| | HSA (%) | Sucrose (%) | Sodium chloride (%) | 2U | 1.54U |
| 1 | 1 | 1 | 0.4 | 0.8 | 0.7 |
| 2 | 1 | 1 | 0.5 | 0.8 | 0.7 |
| 3 | 1 | 1 | 0.6 | 0.7 | 0.7 |
| 4 | 1 | 1 | 0.7 | 0.8 | 0.9 |
| 5 | 1 | 1 | 0.8 | 1 | 0.9 |
| 6 | 1 | 1 | 0.9 | 0.9 | 0.9 |

### Example 4

In this example, the recombinant botulinum toxin type A protein prepared in Example 1 and the component formulas as shown in Table 4 were used, and the same methods for the preparation, reconstitution, and activity assay of lyophilized formulations as in Example 2 were adopted.

Taking into account the losses due to pipeline adsorption and filter elements during the sterile filtration in the sample filling, and the like, in this example, a filling volume of 1 mL was preferred, with a botulinum toxin protein activity of 120 U/mL, and the composition formulas shown in Table 4 were adopted. Meanwhile, the activity of the botulinum toxin protein of serial Nos. 1-9 prior to lyophilization was investigated after preparation according to the formulas. As can be seen from the data in Table 4, no activity was detected for botulinum toxin proteins when sodium chloride or HSA was not present in the components, indicating that sodium chloride and HSA in the components play a key role in maintaining botulinum toxin protein activity. When the content of other components was the same, and HSA at 0.1% was used, the LD₅₀ value varied significantly compared to that of the HSA content of 0.5% and 1%. However, the LD₅₀ value did not differ much with the HSA content of 0.5% and 1%, indicating that the activity of the botulinum toxin protein after preparation and prior to lyophilization was not significantly affected whether HSA was used at 0.5% or 1%. However, when the HSA content decreased to 0.1%, the activity of the botulinum toxin protein after preparation and prior to lyophilization was significantly reduced. Additionally, when the content of other components was the same, and the content of sodium chloride in the components decreased to 0.3%, the activity of the botulinum toxin protein after preparation and prior to lyophilization was also significantly reduced.

Accordingly, activity detection was performed on the biological composition sample prepared with the component formula of serial No. 1 at different steps, including after freeze thawing once after preparation, after filling, and after lyophilization and reconstitution. Specific detection data are shown in Table 5. As can be seen from the data in Table 5, during the lyophilization experiment, the LD₅₀ value of the sample of serial No. 1 at different lyophilization steps did not change much. Meanwhile, the activity of the botulinum toxin protein before lyophilization was 120 U/mL, and after lyophilization and reconstitution, the activity of the botulinum toxin protein was 97 U/mL. It can be seen that by adopting the composition formula and the lyophilization preparation method of the present invention, the activity of the botulinum toxin protein after lyophilization and reconstitution can be effectively maintained at 80% or more of that before lyophilization, fully demonstrating that the composition and the lyophilized formulation of the present invention have strong stability and can maintain high drug activity. Therefore, it indicates that the botulinum toxin protein composition and the lyophilized formulation product of the present invention can be applicable to expanded or large-scale industrial production, and provide strong technical support for the expansion of the application range of the botulinum toxin protein of the present invention.

**Table 4. Composition lyophilization formula of Example 4 and LD₅₀ values after preparation and prior to lyophilization**

| Serial No. | Lyophilization formula | | | LD₅₀ after preparation |
|---|---|---|---|---|
| | HSA (%) | Sucrose (%) | Sodium chloride (%) | |
| 1 | 0.5 | 1 | 0.9 | 11.8pg/U |
| 2 | 1 | 1 | 0.9 | 11.4pg/U |
| 3 | 0.5 | 2 | 0.6 | 11.8pg/U |
| 4 | 0.5 | 2 | 0.45 | 13.8pg/U |
| 5 | 0.5 | 0.5 | 0.45 | 13.8pg/U |
| 6 | 0.5 | 2 | 0.3 | 16.6pg/U |
| 7 | 0.5 | 2 | 0 | 0pg/U |
| 8 | 0.1 | 2 | 0.6 | 17.3pg/U |
| 9 | 0 | 2 | 0.6 | 0pg/U |

**Table 5. Detection results of sample activity at different steps during lyophilization experiment**

| Sampling time | LD50 | Converted into potency |
|---|---|---|
| After freeze thawing once after preparation and filtration | 13.1pg/U | 108U/mL |
| Sampling after filling | 13 .2pg/U | 107U/mL |
| After lyophilization and reconstitution | 14.6pg/U | 97U/mL |

### Example 5

In this example, the recombinant botulinum toxin type A protein prepared in Example 1 and the component formulas as shown in Table 6 were used to conduct a formability study. Firstly, solutions were prepared according to the component formulas shown in Table 6, the solutions were filled into 2-mL vials according to the filling specification (0.25 mL), and the vials were put into a vacuum lyophilizer for lyophilization.

The lyophilization comprises the following procedures:
Freezing stage: The lyophilizer was cooled to -45--50 °C for freezing for 120-150 min;
Primary drying stage: The vacuum degree of the lyophilizer was controlled to be 6-12 Pa, the temperature of the cold hydrazine was kept below -45 °C, the temperature was raised to -38 °C, and drying was performed for 15-25 h; subsequently, the vacuum degree of the lyophilizer was controlled to be 6-12 Pa, the temperature of the cold hydrazine was kept below -45 °C, the temperature was raised to -20 °C, and drying was performed for 5-10 h;
Secondary drying stage: The vacuum degree of the lyophilizer was controlled to be 6-12 Pa, the temperature of the cold hydrazine was kept below -45 °C, the temperature was raised to 25 °C, and drying was performed for 5-10 h; subsequently, the vacuum degree of the lyophilizer was controlled to be 1-3 Pa, the temperature of the cold hydrazine was kept below -45 °C, the temperature was raised to 25 °C, and drying was performed for 2-5 h.

**Table 6. Composition lyophilization formulas of Example 5 and formability investigation after lyophilization**

| Serial No. | Lyophilization formula | | | Formability |
|---|---|---|---|---|
| | HSA (%) | Sucrose (%) | Sodium chloride (%) | |
| 1 | 0.5 | 1 | 0.9 | Good appearance and formability |
| 2 | 0.5 | 1 | 0.3 | Unshaped appearance |
| 3 | 0.5 | 4 | 0.9 | Unshaped appearance |

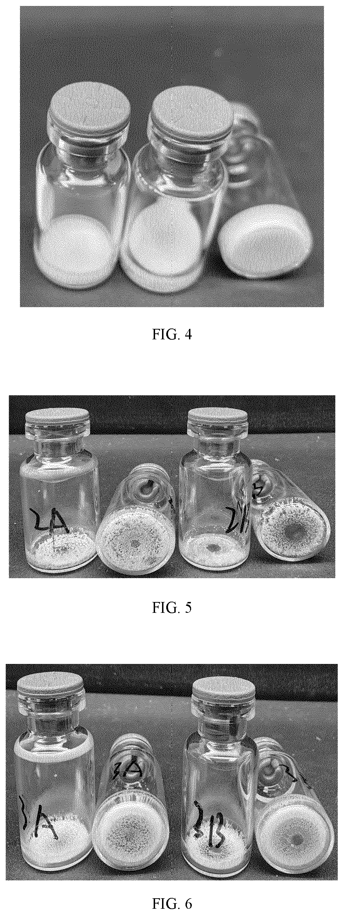

As can be seen from the data in Table 6, different content and proportions of sucrose and sodium chloride in the formulation components lead to different formability of the formulations after lyophilization. The formulation composition of serial No. 1 showed good appearance and formability (see FIG. 4). The formulation composition of serial No. 2, when the NaCl content was reduced from 0.9% to 0.3% in the components, and the concentrations of HSA and sucrose remained unchanged, showed reduced formability after lyophilization (see FIG. 5). According to the general rule, increasing the concentration of the excipient sucrose can improve formability, and therefore the sucrose content in the formulation composition of serial No. 3 was increased to 4%. However, the formability did not improve (see FIG. 6).

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

The foregoing examples and methods described herein can vary based on the abilities, experience, and preferences of those skilled in the art.

The certain order in which the steps of the method are listed in the present invention does not constitute any limitation to the order of the steps of the method.

## Claims

1. A botulinum toxin protein composition, mainly comprising the following components:
a botulinum toxin protein, human serum albumin, a saccharide, and a salt.

2. The composition according to claim 1, wherein the human serum albumin has a mass percentage in the range of 0.2% to 2%;
preferably, the human serum albumin has a mass percentage in the range of 0.5% to 1%.

3. The composition according to claim 1, wherein the saccharide has a mass percentage in the range of 0.5% to 6%;
preferably, the saccharide has a mass percentage in the range of 0.5% to 2%.

4. The composition according to any one of claims 1-3, wherein the saccharide is selected from one or more of trehalose, sucrose, maltose, fructose, raffinose, lactose, and glucose;
preferably, the saccharide is selected from one or more of sucrose and lactose.

5. The composition according to any one of claims 1-3, wherein the salt has a mass percentage in the range of 0.4% to 1%;
preferably, the salt has a mass percentage in the range of 0.4% to 0.9%;
more preferably, the salt has a mass percentage in the range of 0.45% to 0.9%.

6. The composition according to any one of claims 1-3, wherein the salt is selected from any one of an inorganic salt or an organic salt;
preferably, the inorganic salt is selected from one or more of sodium chloride, sodium phosphate, magnesium sulfate, sodium acetate, sodium propionate, and tripotassium phosphate;
preferably, the organic salt is selected from one or more of sodium lactate and sodium succinate;
more preferably, the inorganic salt is sodium chloride.

7. The composition according to claims 1-3, wherein the botulinum toxin protein is a recombinant botulinum toxin protein.

8. The composition according to claim 7, comprising the recombinant botulinum toxin protein, human serum albumin, sucrose, and sodium chloride.

9. The composition according to claim 8, comprising the following components:
the human serum albumin having a mass percentage in the range of 0.2% to 2%,
the sucrose having a mass percentage in the range of 0.5% to 6%, and
the sodium chloride having a mass percentage in the range of 0.4% to 1%;
preferably, the human serum albumin having a mass percentage in the range of 0.5% to 1%,
the sucrose having a mass percentage in the range of 0.5% to 2%, and
the sodium chloride having a mass percentage in the range of 0.4% to 0.9%;
more preferably, the human serum albumin having a mass percentage in the range of 0.5% to 1%,
the sucrose having a mass percentage in the range of 0.5% to 1%, and
the sodium chloride having a mass percentage in the range of 0.45% to 0.9%.

10. The composition according to claim 8 or 9, wherein the recombinant botulinum toxin protein is prepared by cleaving a single-chain polypeptide with a first protease to remove a tag protein, and forming at least one dimer after cleavage with a second protease,
wherein the single-chain polypeptide comprises:
(I) a first polypeptide fragment comprising:
(a) a tag protein;
(b) a structural region comprising a first protease cleavage site; and
(c) a short linker peptide; and
(II) a second polypeptide fragment comprising:
(d) a first functional amino acid structural region comprising a metal ion-dependent protease activity domain;
(e) a structural region comprising a second protease cleavage site; and
(f) a second functional amino acid structural region comprising a receptor-binding domain capable of binding to a surface receptor of a target cell and/or a translocation domain capable of mediating the transfer of the polypeptide across a vesicle membrane, wherein preferably, the first functional amino acid structural region is a light chain of BoNT/A, and the second functional amino acid structural region is a heavy chain of BoNT/A.

11. The composition according to claim 10, wherein the structural region comprising the first protease cleavage site and the structural region comprising the second protease cleavage site are selected from one of or a combination of two of the following enzyme cleavage sites: DDDDK (SEQ ID NO: 12), EXXYXQS (SEQ ID NO: 13), EXXYXQG (SEQ ID NO: 14), HY, YH, or LEVLFQGP (SEQ ID NO: 4);
preferably, the structural region comprising the first protease cleavage site and the structural region comprising the second protease cleavage site are both LEVLFQGP (SEQ ID NO: 4), and the cleavage sites are between Q-G; the single-chain polypeptide sequentially comprises, from the N terminus, a glutathione S-transferase, LEVLFQGPLGS (SEQ ID NO: 15), the light chain of BoNT/A, LEVLFQGP (SEQ ID NO: 4), and the heavy chain of BoNT/A;
preferably, the single-chain polypeptide has an amino acid sequence set forth in SEQ ID NO: 11.

12. A preparation method for the composition according to any one of claims 1-11, wherein the composition is prepared by mixing the components with water and then lyophilizing,
wherein the water is water for injection or normal saline;
the composition is in the form of a lyophilized formulation.

13. A lyophilized formulation comprising a recombinant botulinum toxin protein prepared by the preparation method according to claim 12.

14. A cosmetic method, wherein the method comprises administering the composition according to any one of claims 1-11; preferably, the cosmetic method comprises wrinkle smoothing or prevention, facial slimming, body contouring, and scar repair; more preferably the cosmetic method is for non-therapeutic purposes.

15. A method for preventing and/or treating a disease associated with cholinergic nerves innervating muscles or exocrine glands, wherein the method comprises administering the composition according to any one of claims 1-11,
wherein preferably, the disease associated with cholinergic nerves is selected from:
one or more of Frey's syndrome, crocodile tears syndrome, armpit hyperhidrosis, plantar hyperhidrosis, head and neck hyperhidrosis, body hyperhidrosis, rhinorrhea, Parkinson's disease, amyotrophic lateral sclerosis, sialorrhea, drooling, salivation, spasticity, palatal myoclonus, myoclonus, myokymia, stiffness, benign myospasm, hereditary chin tremor, abnormal mandibular muscle activity, hemimasticatory spasm, hypertrophic branchial myopathy, masseter hypertrophy, tibialis anterior muscle hypertrophy, nystagmus, oscillating vision, supranuclear gaze palsy, epilepsia partialis continua, planned spasmodic torticollis surgery, abductor vocal cord paralysis, refractory mutational dysphonia, upper esophageal sphincter dysfunction, vocal cord granuloma, stuttering, Tourette syndrome, middle ear myoclonus, protective laryngeal closure, speech failure after laryngectomy, protective ptosis, entropion, sphincter of Oddi dysfunction, pseudoachalasia, non-achalasia esophageal dyskinesia, vaginismus, post-operative bed rest, tremor, bladder dysfunction, hemifacial spasm, reinnervation dyskinesia, stiff person syndrome, tetanus, prostatic hyperplasia, obesity treatment, infantile cerebral palsy, achalasia, anal fissure, allergic rhinitis, depression, dental disease, and neuropathic pain.

16. Use of the composition according to any one of claims 1-11 in preparing a medicament for preventing and/or treating a disease associated with cholinergic nerves innervating muscles or exocrine glands,
or
in preparing a medicament for medical cosmetology,
wherein preferably, the medical cosmetology comprises wrinkle smoothing or prevention, facial slimming, body contouring, and scar repair;
preferably, the disease associated with cholinergic nerves is selected from:
one or more of Frey's syndrome, crocodile tears syndrome, armpit hyperhidrosis, plantar hyperhidrosis, head and neck hyperhidrosis, body hyperhidrosis, rhinorrhea, Parkinson's disease, amyotrophic lateral sclerosis, sialorrhea, drooling, salivation, spasticity, palatal myoclonus, myoclonus, myokymia, stiffness, benign myospasm, hereditary chin tremor, abnormal mandibular muscle activity, hemimasticatory spasm, hypertrophic branchial myopathy, masseter hypertrophy, tibialis anterior muscle hypertrophy, nystagmus, oscillating vision, supranuclear gaze palsy, epilepsia partialis continua, planned spasmodic torticollis surgery, abductor vocal cord paralysis, refractory mutational dysphonia, upper esophageal sphincter dysfunction, vocal cord granuloma, stuttering, Tourette syndrome, middle ear myoclonus, protective laryngeal closure, speech failure after laryngectomy, protective ptosis, entropion, sphincter of Oddi dysfunction, pseudoachalasia, non-achalasia esophageal dyskinesia, vaginismus, post-operative bed rest, tremor, bladder dysfunction, hemifacial spasm, reinnervation dyskinesia, stiff person syndrome, tetanus, prostatic hyperplasia, obesity treatment, infantile cerebral palsy, achalasia, anal fissure, allergic rhinitis, depression, dental disease, and neuropathic pain.
